(19) Europäisches Patentamt
European Patent Office
Office européen des brevets

(11) EP 4 549 622 A1

(12) EUROPEAN PATENT APPLICATION

(43) Date of publication:
**07.05.2025 Bulletin 2025/19**

(21) Application number: **24207998.6**

(22) Date of filing: **22.10.2024**

(51) International Patent Classification (IPC):
***C25B 1/04*** *(2021.01)* ***C25B 15/08*** *(2006.01)*
***C01B 4/00*** *(2006.01)* ***C07B 59/00*** *(2006.01)*
***C07C 29/141*** *(2006.01)* ***C07C 29/145*** *(2006.01)*
***C07C 29/149*** *(2006.01)* ***C07C 209/50*** *(2006.01)*
***C07D 211/00*** *(2006.01)*

(52) Cooperative Patent Classification (CPC):
(C-Sets available)
**C25B 1/04; C01B 4/00; C07B 35/02; C07B 59/001; C07C 29/141; C07C 29/145; C07C 29/149; C07C 209/50; C25B 15/081;** C07B 2200/05; C07C 2601/04; C07C 2601/14 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.10.2023 EP 23206885**

(71) Applicant: **BASF SE**
**67056 Ludwigshafen am Rhein (DE)**

(72) Inventors:
- **Vossen, Marcus**
**67117 Limburgerhof (DE)**
- **Stock, Christoph**
**67056 Ludwigshafen am Rhein (DE)**
- **Ernst, Martin**
**67056 Ludwigshafen am Rhein (DE)**
- **Harhausen, Sina**
**67056 Ludwigshafen am Rhein (DE)**
- **Xiong, Jiawen**
**67056 Ludwigshafen am Rhein (DE)**
- **Hueffer, Stephan**
**67063 Ludwigshafen (DE)**
- **Krueger, Marco**
**67056 Ludwigshafen am Rhein (DE)**
- **Weiss, Thomas**
**67056 Ludwigshafen am Rhein (DE)**

(74) Representative: **BASF IP Association**
**BASF SE**
**GBI - Z078**
**67056 Ludwigshafen (DE)**

# (54) HYDROGENATION OF CARBONYL COMPOUNDS USING HYDROGEN WITH LOW DEUTERIUM CONTENT

(57) A process for the hydrogenation of carbonyl compounds consisting of the following steps:
(a) providing hydrogen with a molar share of deuterium ≤ 100 ppm, based on the total hydrogen content, by electrolysis of water using electrical power generated at least in part from non-fossil energy,
(b) hydrogenating a carbonyl compound using the hydrogen provided in step (a) to form the corresponding hydrogenation product, wherein at least one carbonyl group of the carbonyl compound is hydrogenated.

EP 4 549 622 A1

(52) Cooperative Patent Classification (CPC): (Cont.)

C-Sets
**C07C 29/141, C07C 31/10;**
**C07C 29/141, C07C 31/12;**
**C07C 29/141, C07C 31/125;**
**C07C 29/141, C07C 31/20;**
**C07C 29/141, C07C 31/22;**
**C07C 29/141, C07C 33/02;**
**C07C 29/141, C07C 33/025;**
**C07C 29/145, C07C 33/05;**
**C07C 29/145, C07C 33/20;**
**C07C 29/149, C07C 31/08;**
**C07C 29/149, C07C 31/20;**
**C07C 29/149, C07C 33/14**

## Description

The present invention relates to a hydrogenation process for compounds containing carbonyl functionalities. The educt carbonyl compounds contains at least one carbonyl functionality and are preferably selected from one or more of the compound classes of aldehydes, ketones, carboxylic acids, carboxylate esters, carboxylic acid anhydrides, carboxylic acid amides and amino acids. The obtained products of the hydrogenation contain at least one functionality selected from an alcohol, an ether, an alkyl, an alkylene and/or an amino alcohol functionality, depending on the functional groups in the starting material. The obtained products have a lower deuterium content, based on the total hydrogen content, compared to corresponding products generated by reaction with hydrogen that is produced from petrochemical processes using natural gas, condensate or petroleum oil. The invention further relates to the products obtained thereby as well as to their use.

In the hydrogenation of carbonyl compounds, hydrogen and in most cases a catalyst is used.

In modern industrial processes, a significant amount of the hydrogen is provided by steam reforming, thus, from natural gas. However, the petrochemical steam reforming process has its negative impacts with regard to its carbon footprint including the consumption of a lot of fossil-based natural resources and energy.

It is therefore an objective of the present invention to provide environmentally friendly products in an environmentally friendly process for making the same, that process using as little fossil-based energy as possible.

The object is achieved by a process for the hydrogenation of a carbonyl compound comprising the following steps:

(a) providing hydrogen with a molar share of deuterium ≤ 100 ppm, based on the total hydrogen content, by electrolysis of water using electrical power generated at least in part from non-fossil energy,

(b) hydrogenating the carbonyl compound using the hydrogen provided in step (a) to form a hydrogenation product thereof, wherein at least one carbonyl group of the carbonyl compound is hydrogenated.

The molar share of deuterium in hydrogen and downstream compounds based on hydrogen is given in the present application in ppm, based on the total hydrogen content, which is the mol-ppm content of deuterium, based on the total hydrogen content (in hydrogen or in the compounds discussed, respectively).

The deuterium content of hydrogen and downstream compounds based on hydrogen is given in the present application in atom-ppm based on the total molar hydrogen content (total atoms of protium $^1H$ and deuterium $^2H$). The terms "deuterium content" and "molar share of deuterium" are used synonymously throughout the application.

In physical organic chemistry, a kinetic isotope effect is the change in the reaction rate of a chemical reaction when one of the atoms in the reactants is replaced by one of its isotopes. Formally, it is the ratio of rate constants $k_L$ / $k_H$ for the reactions involving the light ($k_L$) and the heavy ($k_H$) isotopically substituted reactants (isotopologues). This change in reaction rate is a quantum mechanical effect that primarily results from heavier isotopologues having lower vibrational frequencies compared to their lighter counterparts. In most cases, this implies a greater energetic input needed for heavier isotopologues to reach the transition state, and consequently a slower reaction rate.

Isotopic rate changes are most pronounced when the relative mass change is greatest, since the effect is related to vibrational frequencies of the affected bonds. For instance, changing a hydrogen atom (H) to its isotope deuterium (D) represents a 100 % increase in mass, whereas in replacing $^{12}C$ with $^{13}C$, the mass increases by only 8 percent. The rate of a reaction involving a C-H bond is typically 6-10 times faster than the corresponding C-D bond, whereas a $^{12}C$ reaction is only 4 percent faster than the corresponding $^{13}C$ reaction.

A primary kinetic isotope effect may be found when a bond to the isotope atom is being formed or broken. A secondary kinetic isotope effect is observed when no bond to the isotope atom in the reactant is broken or formed. Secondary kinetic isotope effects tend to be much smaller than primary kinetic isotope effects; however, secondary deuterium isotope effects can be as large as 1.4 per deuterium atom.

Step (a)

Step (a) concerns the electrolysis of water using electrical power generated at least in part from non-fossil energy.

Electrolysis of water is an environmentally friendly method for production of hydrogen because it uses renewable $H_2O$ and produces only pure oxygen as by-product. Additionally, water electrolysis utilizes direct current (DC) from sustainable energy resources, for example solar, wind, hydropower and biomass.

It is observed that by electrolysis of water, the deuterium atom content of the hydrogen is lower than in the hydrogen generated petrochemically, for example as contained in synthesis gas, in general ≤ 100 ppm, preferably in general ≤ 90 ppm, for example from 30 to 75 ppm. The deuterium atom content in electrolytically produced hydrogen may be as low as 10 ppm. The deuterium is mainly present in the form of D-H rather than $D_2$.

One suitable water electrolysis process is alkaline water electrolysis. Hydrogen production by alkaline water

electrolysis is a well-established technology up to the megawatt range for a commercial level. In alkaline water electrolysis initially at the cathode side two water molecules of alkaline solution (KOH/NaOH) are reduced to one molecule of hydrogen ($H_2$) and two hydroxyl ions (OH-). The produced $H_2$ emanates from the cathode surface in gaseous form and the hydroxyl ions (OH-) migrate under the influence of the electrical field between anode and cathode through the porous diaphragm to the anode, where they are discharged to half a molecule of oxygen ($O_2$) and one molecule of water ($H_2O$). Alkaline electrolysis operates at lower temperatures such as 30-80°C with alkaline aqueous solution (KOH/NaOH) as the electrolyte, the concentration of the electrolyte being about 20% to 30 %. The diaphragm in the middle of the electrolysis cell separates the cathode and anode and also separates the produced gases from their respective electrodes, avoiding the mixing of the produced gases. However, alkaline electrolysis has negative aspects such as limited current densities (below 400 mA/$cm^2$), low operating pressure and low energy efficiency.

In one preferred embodiment of the inventive process, hydrogen is provided by polymer electrolyte membrane water electrolysis. Variants of polymer electrolyte membrane water electrolysis are proton exchange membrane water electrolysis (PEMWE) and anion exchange membrane water electrolysis (AEMWE).

PEM water electrolysis was developed to overcome the drawbacks of alkaline water electrolysis. PEM water electrolysis technology is similar to the PEM fuel cell technology, where solid polysulfonated membranes (Nation®, fumapem®) are used as an electrolyte (proton conductor). These proton exchange membranes have many advantages such as low gas permeability, high proton conductivity ($0.1 \pm 0.02$ S $cm^{-1}$), low thickness (20-300 μm), and allow high-pressure operation. In terms of sustainability and environmental impact, PEM water electrolysis is one of the most favorable methods for conversion of renewable energy to highly pure hydrogen. PEM water electrolysis has great advantages such as compact design, high current density (above 2 A $cm^{-2}$), high efficiency, fast response, operation at low temperatures (20-80°C) and production of ultrapure hydrogen. The state-of-the-art electrocatalysts for PEM water electrolysis are highly active noble metals such as Pt/Pd for the hydrogen evolution reaction (HER) at the cathode and $IrO_2$/$RuO_2$ for the oxygen evolution reaction (OER) at the anode.

One of the largest advantages of PEM water electrolysis is its ability to operate at high current densities. This can result in reduced operational costs, especially for systems coupled with very dynamic energy sources such as wind and solar power, where sudden spikes in energy output would otherwise result in uncaptured energy. The polymer electrolyte allows the PEM water electrolyzer to operate with a very thin membrane (ca. 100-200 μm) while still allowing high operation pressure, resulting in low ohmic losses, primarily caused by the conduction of protons across the membrane (0.1 S/cm), and a compressed hydrogen output.

The PEM water electrolyzer utilizes a solid polymer electrolyte (SPE) to conduct protons from the anode to the cathode while insulating the electrodes electrically. Under standard conditions the enthalpy required for the formation of water is 285.9 kJ/mol. One portion of the required energy for a sustained electrolysis reaction is supplied by thermal energy and the remainder is supplied through electrical energy.

The half reaction taking place on the anode side of a PEM water electrolyzer is commonly referred to as the Oxygen Evolution Reaction (OER). Here the liquid water reactant is supplied to a catalyst where it is oxidized to oxygen, protons and electrons.

The half reaction taking place on the cathode side of a PEM water electrolyzer is commonly referred to as the Hydrogen Evolution Reaction (HER). Here the protons that have moved through the membrane are reduced to gaseous hydrogen.

PEMs can be made from either pure polymer membranes or from composite membranes, where other materials are embedded in a polymer matrix. One of the most common and commercially available PEM materials is the fluoropolymer PFSA, or Nafion®, a DuPont product. While Nafion® is an ionomer with a perfluorinated backbone like Teflon, there are many other structural motifs used to make ionomers for proton-exchange membranes. Many use polyaromatic polymers, while others use partially fluorinated polymers.

An overview over hydrogen production by PEM water electrolysis is given in S. Kumar and V. Himabindu, Material Science for Energy Technologies 2 (2019), pp. 4442 - 4454.

An overview over hydrogen production by anion exchange membrane water electrolysis is given in H. A. Miller et al., Sustainable Energy Fuels, 2020, 4, pp. 2114 - 2133.

K. Harada et al., International Journal of Hydrogen Energy 45 (2020), pp. 31389 - 31 395 report a deuterium depletion by a factor from 2 to 3 in polymer electrolyte membrane water electrolysis. The separation factor β

$$\beta = ([H]/[D])_{gas} / ([H]/[D])_{liquid}$$

where "gas" is the evolved gas and "liquid" is water before the electrolysis was found to be between 2 and 3 at current densities of from 1.0 to 2.0 A $cm^{-2}$, corresponding to a stoichiometric number A of between 4 and 9 at the given water mass flow in the anode. The stoichiometric number A is defined as follows:

$$\lambda = V \times \rho / (J/2F \times 60 \times M_{H2O})$$

where V (mL $min^{-1}$) is the water mass flow in the anode, F is the Faraday constant, J is electrolysis current (A), ρ is the density of water (g $mL^{-1}$) and $M_{H2O}$ (g $mol^{-1}$) is the molar weight of water. A stoichiometric number A of 10 means that 10 times the amount of fresh water than can be theoretically consumed by electrolysis at the given electrolysis current is supplied to the anode.

H. Sato et al., International Journal of Hydrogen Energy 46 (2021), pp. 33 689 - 33 695, report for anion exchange membrane water electrolysis that deuterium concentration in the evolving hydrogen gas is diluted by approximately 1/5 against the feed water, at A = 4.

Hence, deuterium in the evolving hydrogen gas can easily be depleted by a factor of from 2 to 5 with regard to feed water in polymer electrolyte membrane water electrolysis. Depending on the electrolysis conditions (water flow, current density), even higher depletion factors are possible. Since the average deuterium content of water is about 150 ppm, based on the total hydrogen content, hydrogen provided in step (a) of the inventive process may have a deuterium content of from 30 to 75 ppm, based on the total hydrogen content, or even lower.

The electrical power is generated at least in part from non-fossil, renewable resources. In other words, part of the electrical power can still be produced from fossil fuels, preferably from natural gas, since combustion of natural gas causes much lower carbon dioxide emission per Megajoule of electrical energy produced than combustion of coal. However, the portion of electrical energy produced from fossil fuels should be as low as possible, preferably ≤ 50%, preferably ≤ 30%, most preferably ≤ 20%.

The electrical power from non-fossil resources used in water electrolysis according to the invention can be generated by nuclear energy. Nuclear energy is considered renewable by the European Commission, as long as certain preconditions (i. a. safe long-term storage of nuclear waste) are fulfilled.

The electrical power from non-fossil resources used in water electrolysis according to the invention is preferably generated from wind power, solar energy, biomass, hydropower and geothermal energy.

In one preferred embodiment of the inventive process, the electrical power used in water electrolysis is generated from hydropower. There are many forms of hydropower. Traditionally, hydroelectric power comes from constructing large hydroelectric dams and reservoirs. Small hydro systems are hydroelectric power installations that typically produce up to 50 MW of power. They are often used on small rivers or as a low-impact development on larger rivers. Run-of-the-river hydroelectricity plants derive energy from rivers without the creation of a large reservoir. The water is typically conveyed along the side of the river valley (using channels, pipes and/or tunnels) until it is high above the valley floor, whereupon it can be allowed to fall through a penstock to drive a turbine.

Wave power, which captures the energy of ocean surface waves, and tidal power, converting the energy of tides, are two forms of hydropower with future potential.

In one further preferred embodiment of the inventive process, the electrical power used in electrolysis is generated at least in part from geothermal energy. Geothermal energy is the heat that comes from the sub-surface of the earth. It is contained in the rocks and fluids beneath the earth's crust and can be found as far down to the earth's hot molten rock, magma.

To produce power from geothermal energy, wells are dug a mile deep into underground reservoirs to access the steam and hot water there, which can then be used to drive turbines connected to electricity generators. There are three types of geothermal power plants; dry steam, flash and binary. Dry steam is the oldest form of geothermal technology and takes steam out of the ground and uses it to directly drive a turbine. Flash plants use high-pressure hot water into cool, low-pressure water whilst binary plants pass hot water through a secondary liquid with a lower boiling point, which turns to vapor to drive the turbine.

In one further preferred embodiment of the inventive process, the electrical power used in water electrolysis is generated from wind power. Wind power can be used to run wind turbines. Modern utility-scale wind turbines range from around 600 kW to 9 MW of rated power. The power available from the wind is a function of the cube of the wind speed, so as wind speed increases, power output increases up to the maximum output for the particular turbine. Areas where winds are stronger and more constant, such as offshore and high-altitude sites, are preferred locations for wind farms.

In one further preferred embodiment of the inventive process, the electrical power used in water electrolysis is generated from solar power, particularly preferred from photovoltaic systems. A photovoltaic system converts light into electrical direct current (DC) by taking advantage of the photoelectric effect. Concentrated solar power (CSP) systems use lenses or mirrors and tracking systems to focus a large area of sunlight into a small beam. CSP-Stirling has by far the highest efficiency among all solar energy technologies.

In one further preferred embodiment of the inventive process, the electrical power used in water electrolysis is generated from biomass. Biomass is biological material derived from living, or recently living organisms. It most often refers to plants or plant-derived materials which are specifically called lignocellulosic biomass. As an energy source, biomass can either be used directly via combustion to produce heat or electricity, or indirectly after converting it to various forms of biofuel. Conversion of biomass to biofuel can be achieved by different methods which are broadly classified into:

thermal, chemical, and biochemical methods. Wood was the largest biomass energy source as of 2012; examples include forest residues - such as dead trees, branches and tree stumps -, yard clippings, wood chips and even municipal solid waste. Industrial biomass can be grown from numerous types of plants, including miscanthus, switchgrass, hemp, corn, poplar, willow, sorghum, sugarcane, bamboo, and a variety of tree species, ranging from eucalyptus to oil palm (palm oil).

Plant energy is produced by crops specifically grown for use as fuel that offer high biomass output per hectare with low input energy. The grain can be used for liquid transportation fuels while the straw can be burned to produce heat or electricity. Biomass can be converted to other usable forms of energy such as methane gas or transportation fuels such as ethanol and biodiesel. Rotting garbage, and agricultural and human waste, all release methane gas - also called landfill gas or biogas. Crops, such as corn and sugarcane, can be fermented to produce the transportation fuel, ethanol. Biodiesel, another transportation fuel, can be produced from left-over food products such as vegetable oils and animal fats.

Step (b)

In step (b), a carbonyl compound using the hydrogen provided in step (a) is hydrogenated to form a hydrogenation product of the carbonyl compound, wherein at least one carbonyl group of the carbonyl compound is hydrogenated.

In general, the educt carbonyl compound contains at least one carbonyl functionality and is selected from one or more of the compound classes of aldehydes, ketones, carboxylic acids, carboxylic acid esters, carboxylic acid anhydrides, carboxylic acid amides and amino acids. The obtained products of the hydrogenation contain in general at least one functionality selected from an alcohol, an ether, a carboxylic acid ester, an alkyl, an alkylene and/or an amino alcohol functionality, depending on the functional groups in the educt carbonyl compound.

In organic chemistry, a carbonyl group is a functional group with the formula C=O, composed of a carbon atom double-bonded to an oxygen atom, and it is divalent at the C atom. It is common to several classes of organic compounds (such as aldehydes, ketones and carboxylic acids), as part of many larger functional groups (such as carboxylate ester, carboxylic acid anhydride, carboxylic acid amide and/or amino acids). A compound containing a carbonyl group is often referred to as a carbonyl compound. For more information see Saul Patai, ed. (1966). The Carbonyl Group. PATAI'S Chemistry of Functional Groups. Vol. 1. John Wiley & Sons. Jacob Zabicky, ed. (1970). The Carbonyl Group. PATAI'S Chemistry of Functional Groups. Vol. 2. John Wiley & Sons.

The carbonyl compounds to be hydrogenated according to the present invention can have a linear or a cyclic structure.

The hydrogenation product of the reaction between the carbonyl compound and hydrogen depends on the functional group of the starting material.

In general, the hydrogenation (b) of the carbonyl compound is selected from:

(1) hydrogenation of an aldehyde to give a primary alcohol;
(2) hydrogenation of a ketone to give a secondary alcohol;
(3) hydrogenation of a carboxylic acid to give a primary alcohol;
(4) hydrogenation of a carboxylate ester to give a primary, secondary or tertiary alcohol;
(5) hydrogenation of an acid anhydride to give a primary alcohol;
(6) hydrogenation of a cyclic anhydride to a give a lactone and/or a cyclic ether;
(7) hydrogenation of an amide to give an amine.

For an overview on the reactions described above see Rylander, P. Catalytic Hydrogenation in Organic Synthesis, Elsevier, Amsterdam 1979, Chapter 4 - Hydrogenation of Acids, Esters, Lactones and Anhydrides, Chapter 5 - Hydrogenation of Aldehydes, Chapter 6 - Hydrogenation of Ketones, and C.Stock (2023) Hydrogenation and Dehydrogenation; in Ullmann's Encyclopedia of Industrial Chemistry (https://doi.org/10.1002/14356007.a13_487.pub3).

Preferred carbonyl compounds to be hydrogenated in step (b) contain a total of from 2 to 20 carbon atoms and from 1 to 3 carbonyl groups.

Preferred carbonyl compounds can contain from 1 to 3 carbon-carbon double bonds. Examples are citral (two C-C double bonds) and citronellal (one C-C double bond) or unsaturated fatty acids or unsaturated fatty acid ester.

Preferred carbonyl compounds can contain from 1 to 3 further functional groups, preferably selected form amino groups or alcohol groups. Examples are 3-hydroxy-2,2-dimethylpropanal, 2,2-bis-hydroxymethylbutanal and amino acids.

Preferred carbonyl compounds can contain cycloaliphatic or aromatic groups having 4, 5 or 6 ring atoms. Examples are tetramethylcyclobutanedion (4 ring atoms), furfual (5 ring atoms), maleic acid anhydride (5 ring atoms), cyclohexane-1-4-dicarboxylic acid (6 ring atoms) and 4-(2-methylpropyl)acetophenone (6 ring atoms; aromatic).

Double bonds, if present, can be hydrogenated along with the carbonyl groups. Examples are the hydrogenation of maleic acid anhydride to give tetrahydrofurane or of maleic acid ester to give butanediol.

Of major importance is the hydrogenation of alkanals having from 3 to 15 carbon atoms obtainable from oxo-synthesis (hydroformylation), to the corresponding alkanols. Of major importance is also the hydrogenation of fatty acid alkyl esters (preferably the methyl esters) obtainable by transesterification from triglycerides, to the corresponding fatty alcohols. Such fatty acid esters are in general esters of $C_4$-$C_{20}$ fatty acids, which may be saturated or ethylenically unsaturated having 1 to 4 C-C double bonds. Of major importance is also the hydrogenation of ethylenically unsaturated aldehydes resulting from an aldol condensation reaction to give the saturated alcohols. Examples are the aldol condensation products of butyraldehyde and valeraldehyde.

The process of hydrogenating carbonyl compounds can be carried out in various ways, which can be differentiated according to the type of catalyst, the feedstock and the physical state of the reactants.

Catalysts

With rare exceptions, hydrogen is unreactive toward organic compounds in the absence of metal catalysts. The unsaturated substrate is "chemisorbed" onto the catalyst, with most sites covered by the substrate. In heterogeneous catalysts used for hydrogenation, hydrogen forms surface hydrides (M-H) from which hydrogens can be transferred to the chemisorbed substrate. Platinum, palladium, rhodium, and ruthenium form highly active catalysts, which operate at lower temperatures and lower pressures of hydrogen. Non-precious metal catalysts, especially those based on nickel, copper and cobalt are alternatives based on transition metals. The trade-off is activity (speed of reaction) vs. cost of the catalyst and cost of the apparatus required for use of high pressures. Examples are described by C. F. H. Allen and James VanAllan (1955): m-Toylybenzylamine" Organic Syntheses.; Collective Volume, vol. 3, p. 827 and A. B. Mekler, S. Ramachandran, S. Swaminathan, and Melvin S. Newman (1973): "2-Methyl-1,3-Cyclohexanedione". Organic Syntheses.; Collective Volume, vol. 5, p. 743

Catalysts are usually classified into two broad classes: homogeneous and heterogeneous catalysts. Homogeneous catalysts dissolve in the solvent that contains the substrate. Heterogeneous catalysts are solids that are suspended in the same solvent with the substrate or are treated with gaseous substrate. A review of catalysts used for hydrogenation is given in C. Stock (2023) Hydrogenation and Dehydrogenation; Chapter 1.3: "Catalysts" in Ullmann's Encyclopedia of Industrial Chemistry (https://doi.org/10.1002/14356007.a13_487.pub3).

Heterogeneous catalysts are more common industrially for hydrogenation of carbonyl compounds.

Heterogenous catalysts consist of one or more active metals in active pure form or on a carrier/support. Additionally, additives are used to increase activity, selectivity and/or stability. See Gallei, E.F., Neumann, H.-P., (1994), Chemie Ingenieur Technik, 7, 924-928 for more background information.

Active metals for the catalysis of hydrogenation reactions are typically precious metals such as palladium, platinum and ruthenium or other transition metals like nickel, copper, zinc and cobalt. Often, a combination of two or more metals is used, or one element is used as an additive for another. In this case, the metals can exist separately on the surface of the catalyst or as intermetallic phases. The latter might show significantly different properties compared to the individual elements as published by Armbrüster, M. (2020) in Science and Technology of advanced Materials, 21, 303-322.

Heterogenous catalysts usually consist of an active metal supported on a carrier material. Carrier materials can be carbon or inorganic oxides of silicon, aluminum, zirconium or titanium like $SiO_2$, $Al_2O_3$, $ZrO_2$ or $TiO_2$. Alternatively, polymers and metal structures can be used as carrier material. The catalyst carrier has to be stable under the reaction conditions, i.e. not changing its physical or chemical properties.

In industry, precious metal hydrogenation catalysts are deposited from solution on the support, which is a cheap, bulky, porous, usually granular material, such as activated carbon, alumina, calcium carbonate or barium sulfate. For example, platinum on carbon is produced by reduction of chloroplatinic acid in situ in carbon. Examples of these catalysts are 5% ruthenium on activated carbon, or 1% platinum on alumina. See "Platinum Heterogeneous Catalysts - Alfa Aesar (www.alfa.com: Archived from the original on 18 January 2018. Retrieved 28 April 2018).

Some exceptional base metal catalysts, such as Raney nickel, are typically much cheaper and do not need a support. Also, in the laboratory, unsupported (massive) precious metal catalysts such as platinum black are still used, despite the cost.

As in homogeneous catalysts, the activity is adjusted through changes in the environment around the metal, i.e. the coordination sphere. Different faces of a crystalline heterogeneous catalyst display distinct activities, for example.

This can be modified by mixing metals or using different preparation techniques. Similarly, heterogeneous catalysts are affected by their supports.

Most preferred are the following hydrogenation reactions (1) to (23) carried out in step (b):

(1) Hydrogenation of citral to give geraniol/nerol

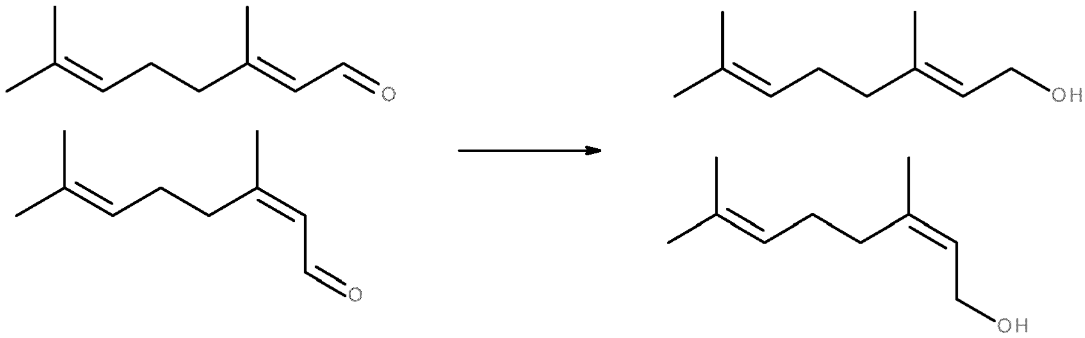

The hydrogenation can be carried out as suspension hydrogenation in batch or continuous mode on ruthenium-based catalysts at e. g. 20 to 80 bar and 50 to 100 °C. The selectivity for the formation of geraniol and nerol can be influenced by selection of the educt compound, reaction conditions and additives. See e. g. WO 2017/211784 and EP 1317959.

(2) Hydrogenation of citronellal to give citronellol

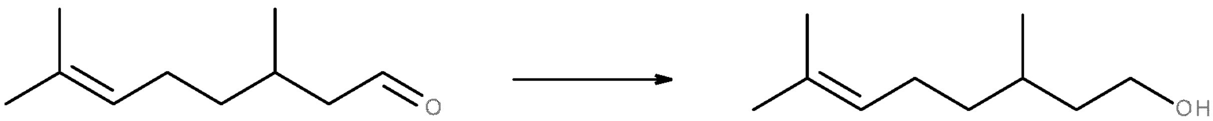

The hydrogenation can be carried out as suspension hydrogenation as described above for the hydrogenation of citronellal. See e.g. DE 10231942, EP 71787 and WO 2017/60243.

(3) Hydrogenation of 3-hydroxy-2,2-dimethylpropanal to give neopentylglycol

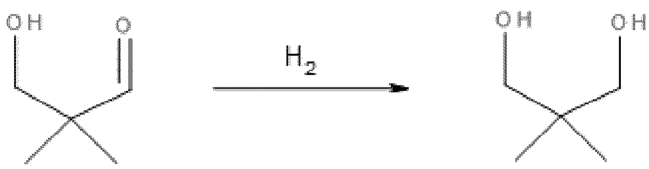


The hydrogenation can be carried out as continuous fixed bed hydrogenation on copper-based catalysts. See e. g. US 2011/0282106 and EP 759896.

(4) Hydrogenation of adipic acid and adipic acid esters to give 1,6-hexanediol

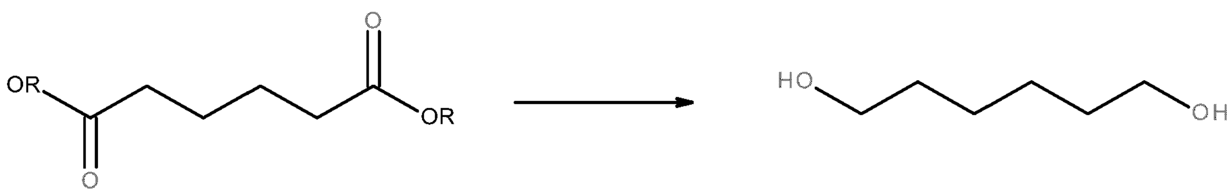

The hydrogenation can be carried out as continuous fixed bed hydrogenation on a copper- or cobalt-based catalysts. See e. g. WO 2010/115759, DE 10253751 and DE 19750532.

(5) Hydrogenation of 2,2-bis-hydroxymethylbutanal to give trimethylolpropane

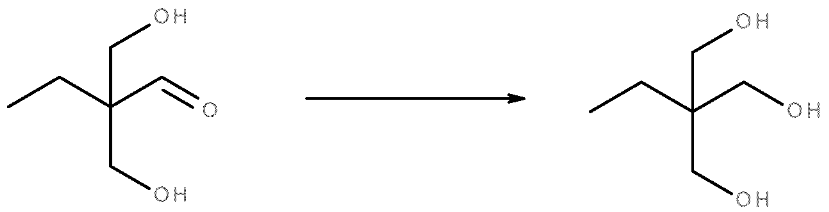

The hydrogenation can be carried out as fixed bed hydrogenation in continuous mode on a copper-based catalyst at 50 to 100 bar and 100 to 150 °C. See e. g. WO 2011/061185.

(6) Hydrogenation of lower alkanals (from hydroformylation) to give lower alkanols (oxo-alkohols)

(6-1) propionaldehyde to give propanol;
(6-2) butyraldehyde to give n-butanol;
(6-3) isobutyraldehyde to give isobutanol;
(6-4) valeraldehyde to give pentanol.

The hydrogenations can be carried out as liquid-phase hydrogenations in continuous fixed bed-mode on copper- or copper/nickel-catalysts at 20 to 50 bar and 100 to 150 °C. Gas phase hydrogenation is also possible. See e. g. EP 3878831, DE 10218848, WO 2013/131936, EP 0759896, WO 2022/250790, WO 2020/190550 and WO 2019/158456.

(7) Hydrogenation of higher alkanals (from hydroformylation) to give higher alkanols (oxo-alcohols)

Hydrogenation of hydroformylation products to give isononanol, tridecanol or C13/C15-alcohol.

(8) Hydrogenation of the aldol condensation products of butyraldehyde and valeraldehyde to give 2-ethylhexanol and 2-propylheptanol

The hydrogenation can be carried out as continuous fixed bed hydrogenation on copper- or cobalt-catalysts in the liquid phase. See DE 100 62 448 and EP 0987240. Apart from the carbonyl function, the double bond resulting from the aldol condensation reaction is also hydrogenated.

(9) Hydrogenation of 4-(2-methylpropyl)acetophenone to give 1-(1-Hydroxyethyl)-4-(2-methylpropyl)-benzene (an Ibuprofen precursor)

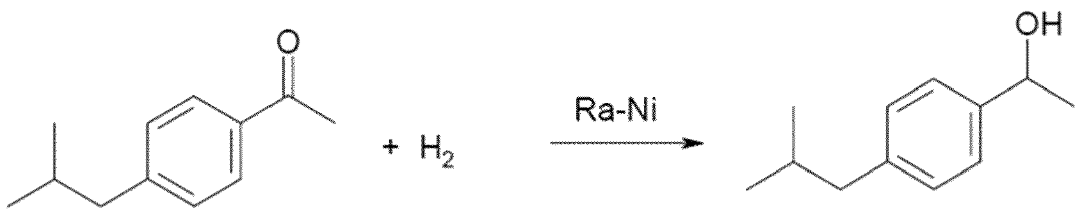


The hydrogenation can be carried out as suspension hydrogenation in batch mode on Raney-nickel at 40 bar and 100 °C or on copper catalysts. See e. g. EP 358420.

(10) Hydrogenation of maleic acid anhydride or maleic acid esters to give tetrahydrofurane (THF) or butanediol (BDO)

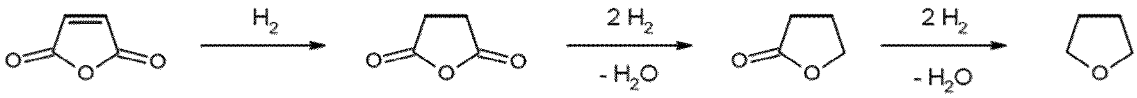


(11) Hydrogenation of cyclohexane-1,4-dicarboxylic acid ester to give cyclohexane-1,4-dimethanol

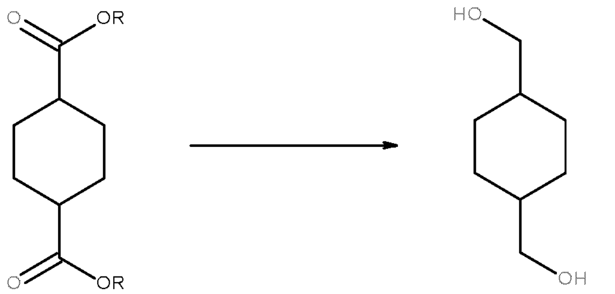

The hydrogenation can be carried out on copper- or copper/chromium-catalysts in the gas phase. See e. g. US 52319129, US 5399742 and US 5387752.

(12) Hydrogenation of amino acids to give amino alcohols

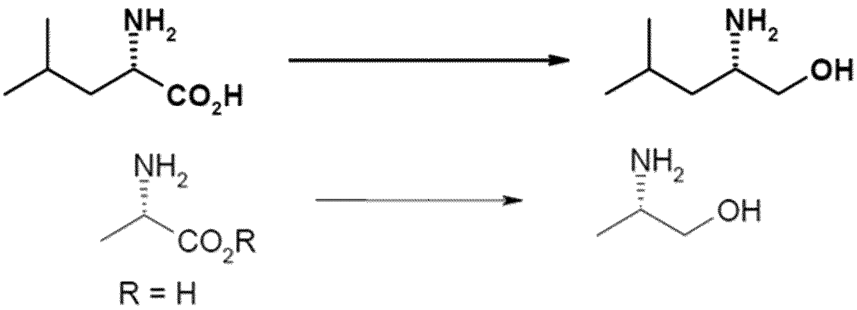

The hydrogenation can be carried out on noble metal catalysts in suspension mode. See e.g. EP 696575 (Bayer/Lanxess), WO 99/38613 (Bayer/Lanxess), EP 1051388 (Bayer/Lanxess) and WO 99/38838 (Bayer/Lanxess).

(13) Hydrogenation of acetone to give isopropanol

The hydrogenation can be carried out as continuous fixed bed hydrogenation on nickel- or copper-catalysts at 90 to 150 °C and 30 to 50 bar. See e. g. US 2009/0093656, EP 1070698 and US 6939995.

(14) Hydrogenation of fatty acid esters to give fatty alcohols

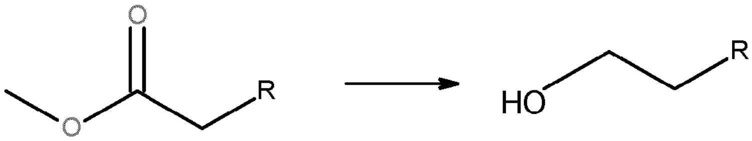

R is preferably $C_2$-$C_{18}$ alkyl or 1- to 4-fold unsaturated alkenyl.

The triglycerides are converted to methyl esters by transesterification. The hydrogenation of the methyl esters can, for example, be carried out in liquid phase on copper-based catalyst at > 200 bar. See e. g. DE 1228603. In the hydrogenation of fatty acid esters different qualities of esters can be used. Unsaturated fatty acid esters may be

hydrogenated into the saturated fatty alcohols or may remain unsaturated.

A further, technically important application of the use of hydrogen with a molar share of deuterium ≤ 100 ppm provided by electrolysis of water using electrical power generated at least in part from non-fossil energy is the hydrogenation of fatty acid esters from plant oils to fatty alcohol mixtures. Fatty alkohols based on renewable resources are available by hydrogenation of vegetable oils like rapeseed oil, sunflower oil, coconut oil, soybean oil, corn oil, palm oil and macauba palm oil, conducting

i) a hydrogenation, preferably a transesterification and a hydrogenation, in particular a trans-esterification followed by a hydrogenation or

ii) a hydrolysis, preferably a hydrolysis and a hydrogenation, in particular a hydrolysis followed by a hydrogenation.

Preferably, the resulting fatty alcohol composition comprises at least 45 wt.-% based on the total weight of $C_{4-22}$ fatty alcohols. Any suitable transesterification method can be conducted. Preferably, the transesterification is conducted in the presence of simple alcohols such as methanol, ethanol, propanol, and/or isopropanol providing glycerol and the respective esters. In a preferred embodiment, the transesterification is conducted in the presence of methanol. The reaction can further comprise the addition of a catalysts. Preferably, the alcohol is provided in excess.

According to EP 4234665 A1, preferably the hydrogenation of Macauba oil fatty acid ester is performed at a temperature of about 50 to about 300 °C, more preferably of about 100 to about 270 °C.

DE 19912684 C disclosed that the final hydrogenation of the alkyl ester fraction based on coconut oil or palm kernel oil fatty acid ester with retention of the double bonds can be carried out in the presence of commercially available zinc/chromium catalysts, at temperatures in the range from 250 to 350 °C and a hydrogen pressure of 200 to 275 bar.

(15) Hydrogenation of tetramethylcyclobutanedion to give tetramethylcyclobutanediol

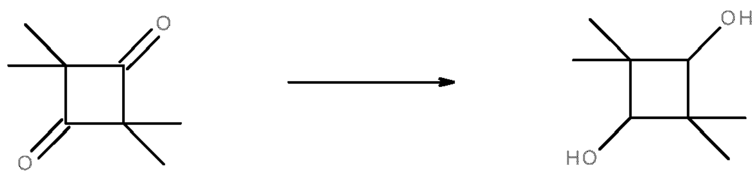

The hydrogenation is carried out by Eastman and described e. g. in US 2008/0132738 and US 9238602.

(16) Hydrogenation of acetic acid to give ethanol

The hydrogenation can be carried out as gas phase hydrogenation on a cobalt-based catalyst at 260 to 300 °C and 80 to 200 bar. See e. g. WO 2011/056595 and US 2011/0282109.

(17) Hydrogenation of furfural to give furfuryl alcohol

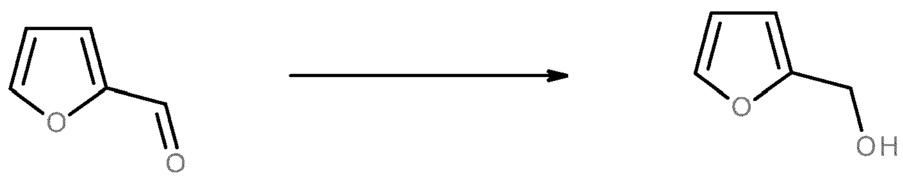

(18) Hydrogenation of homofarnesylsäureisopropylester to homofarnesol

Homofarnesol is an important precursor for the synthesis of ambroxdiol via sclareolid as intermediate.

(19) Hydrogenation of dimethyl terephthalate to 1,4-benzendimethanol

(20) Hydrogenation of levulinic acid methyl ester to 1,4-pentanediol

(21) Hydrogenation of levulinic acid ethyl ester to 1,4-pentanediol

(22) Hydrogenation of levulinic acid butyl ester to 1,4-pentanediol

(23) Hydrogenation of dimethyl adipate to 1,6-hexanediol

The hydrogenation can be carried out in the liquid phase or in the gas phase on copper-based catalysts, e. g. at 140 to 160 °C and 30 to 50 bar. See e. g. Jiminez-Gomez, C.P., Cecilia, J.A., Duran-Martin, D., et al. (2016) J. Catal. 336, 107-115.

The inventive processes of this application and the thus synthesized chemical compounds can be used in the synthesis of surfactants: Carboxylic compounds can be reduced to the corresponding alcohols using the inventive hydrogen. The transformation of fatty acids, fatty acid esters and fatty acid triglycerides can for example be carried out by the procedures described in WO2010133619, WO2011133037, WO2016080552, DE2513377, EP2432756, WO9305003, DE2513377, US3729520 and documents referenced therein. Fatty alcohols, both saturated and unsaturated, can be made by choice of a suitable catalyst known to the person skilled in the art. The choice of catalyst and conditions determine, if and to what degree unsaturated fatty acids are reduced to their saturated forms. Fatty alcohols can for example be made from triglycerides, fatty acid alkyl esters, fatty acids and fatty acid salts using the inventive hydrogen by either gas-phase hydrogenation, trickle-bed hydrogenation or suspension hydrogenation.

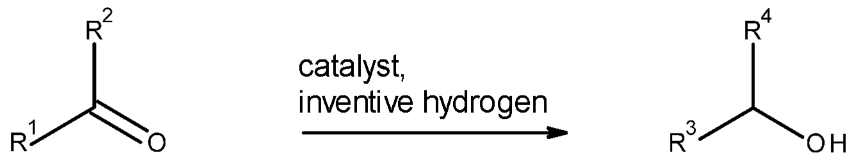


$R^1$ and $R^3$ are organic moieties, $R^2$ can be Hydrogen, an organic moiety, or Hydroxyl, $R^4$ can be an organic moiety or Hydrogen.

The resulting hydroxy-compounds, for example fatty alcohols, can be used to produce surface active compounds, for example surfactants, for example by alkoxylation.

Examples

| Reactant | Product | Conditions |
|---|---|---|
| Rapeseed Oil | Fatty Alcohol mixture | Flow reactor, $CuO_{0.6\text{-}0.85}(Al_2O_3)_{0.1\text{-}0.34}(La_2O_3)_{0.01\text{-}0.2}$, 200 bar hydrogen, 200-220 °C |
| Lauric acid | Lauryl alcohol | Co-Y-Pd-Mo-Catalyst, 240 bar hydrogen, 230 °C, 2 hours |
| oleic acid-methyl ester | oleic alcohol | $ZnO/Al_2O_3$-catalyst, methanol, 250 bar hydrogen, 230 °C |

The inventive product of the inventive hydrogenation process of carbonyl compounds are characterized by the molar share of deuterium in the hydrogen bound in the compounds as a result of step (b) of the process of the invention. This molar share of deuterium is ≤ 100 ppm, preferably in the range of from 10 to 95 ppm, more preferably in the range of from 15 to 90 ppm, most preferably in the range of from 20 to 80 ppm, especially in the range of from 30 to 75 ppm, based on the total content of said hydrogen.

Said low deuterium molar share is introduced by steps (a) and (b) of the process according to the present invention. With the present invention environmentally friendly hydrogenation products and an environmentally friendly process for making the same, wherein said process uses as little fossil energy as possible, are provided.

As mentioned above, it is important that the origin of the hydrogen and down-stream compounds obtained by clean energy can be tracked in a reliable way.

Today, the majority of hydrogen is produced from fossil fuels by steam reforming of natural gas and other light hydrocarbons, partial oxidation of heavier hydrocarbons, and coal gasification. According to the invention, hydrogen obtained by electrolysis is obtained by using non-fossil energy sources. It is expected that the electrification (power generation) of fossil sources will be fully replaced by the generation of power by non-fossil resources in the near future. The downstream products based on hydrogen obtained by electrolysis can be distinguished by their deuterium molar share from hydrogenation products based on hydrogen prepared by petrochemical processes.

The present invention therefore relates to the use of the molar share of deuterium in downstream compounds based on hydrogen for tracing the origin, especially the energetic origin, of the hydrogen bound in the downstream compounds based on hydrogen, wherein the downstream compounds are compounds generated by hydrogenation of

carbonyl compounds.

The present invention further relates to a process for tracing the origin, especially the energetic origin, of hydrogen bound in downstream compounds based on hydrogen by determining the molar share of deuterium in said downstream compounds based on hydrogen, wherein the downstream compounds are compounds generated by hydrogenation of carbonyl compounds.

Tracing is in the meaning of the present invention synonymous with tracking.

The origin is in the meaning of the present invention the preparation method of the hydrogen employed, especially electrolysis and/or the energetic origin, i.e. non-fossil energy sources. As mentioned above, it is expected that the electrification (power generation) of fossil sources will be fully replaced by the generation of power by non-fossil resources in the near future. Hydrogen made by electrolysis is in this case hydrogen of non-fossil origin. Examples for non-fossil power sources are mentioned above.

The inventive process for tracing the origin, especially the energetic origin, of hydrogen and downstream compounds mentioned above may be employed as a single tracing (tracking) method or in combination with further tracing (tracking) methods.

Suitable deuterium molar shares of the compounds generated by hydrogenation of carbonyl compounds are mentioned in the present application.

Furthermore, the products according to the process described herein can be converted to a product as described on page 18, line 29 to page 26, line 29 of European patent application No. EP24164893.0.

As mentioned above, alcohols are one of the preferred chemical classes generated by the hydrogenation of carbonyl compounds. In that regard, it is additionally mentioned that also such alcohols $R\text{-}CH_2OH$ may further be hydrogenated under elimination of methane and water to the respective RH compounds, whereas R can generally mean any carbon containing radical such as substituted or unsubstituted, linear or branched, saturated hydrocarbon radicals. This type of reaction is also known as dehydroxymethylation.

Such hydrogenation reactions in which hydrogen with a molar share of deuterium $\leq$ 100 ppm provided by electrolysis of water using electrical power generated at least in part from non-fossil energy is used for the hydrogenation of alcohols can be used for the preparation of alkanes from fatty alcohols. Typical reaction conditions are, for example, be described in WO 2007/068,371. Therein, a process for the production of linear saturated alkanes from primary alcohols is disclosed, whose C chain contains one carbon atom more than the alkane by reductive dehydroxymethylation of the primary alcohols at temperatures of 100 to 300°C and pressures of 1 to 250 bar in the presence of hydrogen and a catalyst. As primary alcohols, fatty alcohols of natural origin containing 8 to 24 carbon atoms are preferably used.

Examples

I Hydrogen Production

Experimental Setup and Method: Electrolysis cell design and Hydrogen production conditions

1) Polymer Electrolyte Membrane / Proton Exchange Membrane Electrolysis (PEM)

Electrolysis Cell:_Water electrolysis was conducted with a circular commercial PEM electrolysis cell (model ZE 200, Sylatech Analysetechnik GmbH, 0.007 $m^2$ active area). The cell stack was sealed with O-rings and wrapped with heat insulation fabric for isothermal operating conditions. A Nafion® 117 standard membrane (supplier DuPont, dry thickness 180 microns) was assembled by HIAT GmbH with catalytic active coating materials iridium (19 $g/m^2$) and platinum (8 $g/m^2$). Water distribution at the anode half-cell was realized with a titanium mesh. Before the polymer electrolyte membrane, a porous transport layer with sintered titanium fiber material is used for controlled flow while a porous graphite plate was situated on the cathode-side.

Experimental Conditions: Water with controlled temperature was supplied at constant flow of 9.5 g/h to the anode compartment. The cell pressure on cathode and anode side was controlled with PC valves. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic cell-water was re-cycled, whereas on the cathode the separated water was drained. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

2) Alkaline Electrolysis Cell (AEC)

Electrolysis Cell:_Alkaline water electrolysis was conducted with a circular AEC electrolysis cell (model Electro

MP Cell, supplier ElectroCell Europe A/S, 0.01 $m^2$ electrode area). As electrodes Nickel 2.4068 material was used and separated by a commercial standard Zirfon Perl UTP 500 membrane (open mesh polyphenylene sulfide fabric symmetrically coated with a mixture polymer / zirconium oxide; thickness 500 microns; 0.023 $m^2$ active area; supplier Agfa-Gevaert N.V.) in zero-gap cell configuration.

Experimental Conditions: Alkaline water (32 wt% potassium hydroxide, technical standard grade) with controlled temperature was supplied at constant flow of 27.8 kg/h to the anode and cathode compartment. The cell pressure on cathode and anode side was equalized via PC valve control. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic and cathodic cell-water was re-cycled. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

3) Anion Exchange Membrane / Alkaline Electrolyte Membrane Electrolysis (AEM)

Electrolysis Cell:_The AEM experiments were executed in a commercial, fully automated 2.4 kW EL 4.0 cell supplied by Enapter GmbH, 10117 Berlin and a 1 wt% potassium hydroxide (standard grade) electrolyte solution.

Test station:_As recommended by the supplier the EL 4.0 electrolyzer is run with a 1 wt% potassium hydroxide (technical standard grade) solution, hydrogen production at operating conditions (experimental conditions such as temperature and pressure settings see table) was 480 l/h at approx. 400 ml water consumption. The evolved hydrogen gas was treated with desiccant dryers, ensuring complete separation of moisture in the electrolysis gas flow to yield < 0,03 wt% water in the hydrogen gas stream. Small gas samples of the dehydrated cathodic hydrogen gas were taken from the continuous flow with an auto-sampler at regular intervals to conduct hydrogen gas analytics described below.

4) Solid Oxide Electrolysis Cell (SOE)

Solid Oxide cell stacks from Elcogen- Elco Stack (Elcogen OY, Vantaa 01510 Finland), were used and a E3000 unit was operated in reverse mode at 700°C and 35A electrolyzing current; Anode functional composition due to the supplier is NiO/YSZ. Cathode is of LSC type [La(Sr)CoO3]: The principle is also described in Novel high-performance solid oxide fuel cells with bulk ionic conductance dominated thin-film electrolytes - ScienceDirect (www.sciencedirect.com/science/article/pii/S037877531201097X); D. Stover et al, Journal of Power Sources; Volume 218, 15 November 2012, Pages 157-162.

The hydrogen stream was used with no further purification/dryer. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

II Further examples

2,2,6,6-Tetramethyl-4-piperidinol from 2,2,6,6-tetramethyl-4-piperidone

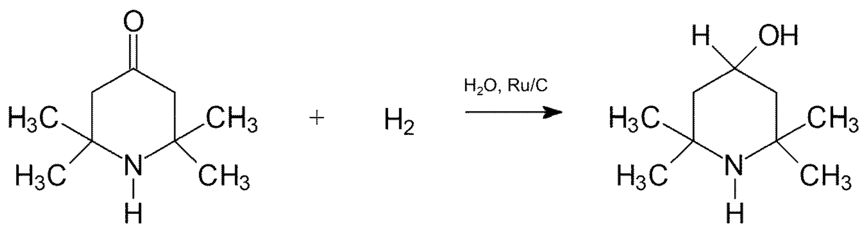


2,2,6,6-Tetramethyl-4-piperidone was reacted with hydrogen in the presence of a ruthenium catalyst under pressure and a maximum of 110°C to produce 2,2,6,6-tetramethyl-4-piperidinol. The temperature in the hydrogenation vessel was kept constant throughout the reaction, and both the reaction and hydrogen dosing were controlled by pressure. The end of the hydrogenation reaction was determined by a decrease in hydrogen uptake.

The results of the hydrogen production are shown in Table 1.

| Electrolyser type | Example I) PEM-electrolyser | Example II) PEM electrolyser | Example III) Alkaline electrolyser | Example IV) Alkaline electrolyser | Example V) AEM | Example VI) AEM | Example VII) Solid oxide |
|---|---|---|---|---|---|---|---|
| Water-type/deuterium content | VSMOW/155,76 ppm D | Selected* surface water/139 ppm D | VSMOV/155,76 ppm D | Selected* surface water/139ppm D | VSMOW/155,76 ppm D | Selected* surface water/139 ppm D | Selected* surface water/139 ppm D |
| Current density [A/cm2] | 1.35 | 1.35 | 0.8 | 0.8 | 1.6 | 1.6 | 0.78 |
| Applied voltage [V] | 1.9 | 1.9 | 2.15 | 2.15 | 1.9 | 1.9 | 1.35 |
| Operating temperature [°C] | 68 | 68 | 81 | 81 | 45 | 45 | 710 |
| Cell pressure [bar] | 23 | 23 | 25 | 25 | 35 | 35 | 1.3 |
| Voltage efficiency [%] | 57 | 57 | 53 | 53 | 66 | 66 | 81 |
| Electrical efficiency [kWh/kg H2] | 52 | 52 | 48.5 | 48.6 | 69 | 69 | 47 |
| Deuterium content in hydrogen [ppm] | 86 | 77 | 95 | 87 | 74 | 66 | 38 |

* Isar river surface water (Munich) collected in winter season January 2021.

Experimental Setup and Method: "D content (Deuterium content) in samples"

The following method descriptions apply for determination of the molar share of deuterium based on the total hydrogen content (Deuterium content) of gas and liquid samples. The isotopic H/D-share analysis is based on mass spectroscopy. Two different methods are used: method A for gas samples and method B for liquid samples.

For determination of the "D content in gas and liquid samples" it is of crucial importance not to contaminate the samples e.g. with ambient humidity or other ambient components containing hydrogen or deuterium. Therefore gas-tight materials and sealings must be used with clean sample containers to avoid any cross-contamination. Therefore, before filling and sealing a sample container it must be flushed at least 20 times the sample container volume with the gas or liquid stream to be analyzed. The same is valid for the experimental setup of the gas sampler and mass spectrometer. Utmost care must be taken to avoid cross-contamination e.g. via condensation of humidity. The analytical setup from sampling to mass spectrometry is validated with known reference samples.

Method A) Gas samples

Total Deuterium from HD and $D_2$ in hydrogen gas samples was determined via ultra-high resolution quadrupole mass spectrometry using a Hiden DLS-20 (Hiden Analytical Ltd., Warrington, Cheshire, UK) analyzer setup. The general method setup is described in C.C. Klepper, T.M. Biewer, U. Kruezi, S. Vartanian, D. Douai, D.L. Hillis, C. Marcus, Extending helium partial pressure measurement technology to JET DTE2 and ITER; Rev. Sci. Instrum., 87 (11) (2016); doi: 10.1063/1.4963713. For the hydrogen gas samples the threshold ionization mass spectrometry mode (TIMS) was used as described in S. Davies, J.A. Rees, D.L. Seymour; Threshold ionization mass spectrometry (TIMS); A complementary quantitative technique to conventional mass resolved mass spectrometry; Vacuum, 101 (2014), pp. 416-422; doi: 10.1016/j.vacuum.2013.06.004. Sensitivity is +/-1 ppm.

Method B) Liquid samples

Analysis of liquid samples (carbonyl compounds and compounds generated by hydrogenation of carbonyl compounds) was executed via isotope ratio monitoring gas chromatography/mass spectrometry (IRMS). Therefore, a DELTA V PLUS CF-IRMS mass spectrometer was used. This mass spectrometer with magnetic sector with continuous flux DELTA V PLUS CF-IRMS is used to measure the isotopic ratio of D/H.

Measurement of D/H in a continuous He-flow mode needs the complete removal of low energy 4 He+ions from the HD+ion beam at m/z 3). The method is described in RAPID COMMUNICATIONS IN MASS SPECTROMETRY Rapid Commun. Mass Spectrom. 13, 1226-1230 (1999), W.A. Brandt et al. Sensitivity is within +/-3 ppm.

Method C) Analysis of isotope ratios in starting materials and products

Analysis of isotope ratios in starting materials and products was furthermore executed via SNIF-NMR related methods ("site-specific natural isotope fractionation studied by nuclear magnetic resonance") using high resolution $^2$H-NMR (Bruker Avance NEO 600 MHz NMR Spectrometer and Bruker Avance III HD 700 MHz NMR Spectrometer both equipped with TCI probes).

Principles of this technology are described in Gérard J. Martin, Serge Akoka, and Maryvonne L. Martin; SNIF-NMR Part 1: Principles; in Graham A. Webb (ed.), Modern Magnetic Resonance, Springer (2008) pp 1651-1658 as well as Maryvonne Martin, Benli Zhang, and Gérard J. Martin; SNIF-NMR Part 2: Isotope Ratios as Tracers of Chemical and Biochemical Mechanistic Pathways; in Graham A. Webb (ed.), Modern Magnetic Resonance, Springer (2008) pp 1659-1667 and Gérard J. Martin, Maryvonne L. Martin and Gérald Remaud; SNIF-NMR Part 3: From Mechanistic Affiliation to Origin Inference; in Graham A. Webb (ed.), Modern Magnetic Resonance, Springer (2008) pp 1669-1680.

II Products generated by hydrogenation of carbonyl compounds

Carbonyl compounds are hydrogenated with or without catalyst in synthesis processes in industrial scale as described above.

## Claims

1. A process for the hydrogenation of carbonyl compounds consisting of the following steps:

   (a) providing hydrogen with a molar share of deuterium ≤ 100 ppm, based on the total hydrogen content, by

electrolysis of water using electrical power generated at least in part from non-fossil energy,
(b) hydrogenating a carbonyl compound using the hydrogen provided in step (a) to form the corresponding hydrogenation product, wherein at least one carbonyl group of the carbonyl compound is hydrogenated.

**2.** The process of claim 1, wherein the electrical power is generated from wind power, solar energy, biomass, hydropower and geothermal energy.

**3.** The process according to claim 1 or 2, wherein hydrogen is provided by polymer electrolyte membrane water electrolysis.

**4.** The process according to claim 3, wherein hydrogen is provided by proton exchange membrane water electrolysis (PEMWE) or anion exchange membrane water electrolysis (AEMWE).

**5.** The process according to any one of claims 1 to 4, wherein the molar share of deuterium in the hydrogen provided in step (a) is in the range of from 10 to 95 ppm, preferably in the range of from 10 to 90 ppm, more preferably in the range of from 20 to 80 ppm, and most preferably in the range of from 30 to 75 ppm, based on the total hydrogen content.

**6.** The process according to any one of claims 1 to 5, wherein step (b) is carried out as heterogeneously or homogeneously catalyzed reaction.

**7.** The process according to any one of claims 1 to 6, wherein in step (b) the hydrogenation of the carbonyl compound is selected from:

(1) hydrogenation of an aldehyde to give a primary alcohol;
(2) hydrogenation of a ketone to give a secondary alcohol;
(3) hydrogenation of a carboxylic acid to give a primary alcohol;
(4) hydrogenation of a carboxylate ester to give a primary, secondary or tertiary alcohol;
(5) hydrogenation of an acid anhydride to give a primary alcohol;
(6) hydrogenation of cyclic anhydrides to give a lactone and/or a cyclic ether;
(7) hydrogenation of an amide to give an amine;

**8.** The process according to any one of claims 1 to 7, wherein the carbonyl compound to be hydrogenated in step (b) contains a total of from 2 to 20 carbon atoms and from 1 to 3 carbonyl groups.

**9.** The process according to claim 8, wherein the carbonyl compound to be hydrogenated in step (b) contains from 1 to 3 carbon-carbon double bonds.

**10.** The process according to claim 8 or 9, wherein the carbonyl compound to be hydrogenated in step (b) contains from 1 to 3 further functional groups, preferably selected from amino groups and alcohol groups.

**11.** The process according to any one of claims 8 to 10, wherein the carbonyl compound to be hydrogenated in step (b) contains a cycloaliphatic or aromatic group having 4, 5 or 6 ring atoms.

**12.** The process according to any one of claims 8 to 11, wherein one or more double bonds, if present, are hydrogenated in step (b) along with the at least one carbonyl group.

**13.** The process according to any one of claims 1 to 10 and 12, wherein in step (b) alkanals having from 3 to 15 carbon atoms obtainable from oxo-synthesis (hydroformylation) are hydrogenated to the corresponding alkanols, or fatty acid alkyl esters obtainable by transesterification from triglycerides are hydrogenated to the corresponding fatty alcohols, or ethylenically unsaturated aldehydes resulting from an aldol condensation reaction are hydrogenated to give the corresponding saturated alcohols.

**14.** Hydrogenation product, obtainable by the process according to any one of claims 1 to 13.

**15.** Hydrogenation product according to claim 14, wherein the molar share of deuterium in the hydrogen bound in the compound as a result of hydrogenation step (b) of the process according to any one of claims 1 to 6 is $\leq$ 100 ppm, preferably in the range of from 10 to 95 ppm, more preferably in the range of from 15 to 90 ppm, most preferably in the range of from 20 to 80 ppm, especially in the range of from 30 to 75 ppm, based on the total content of said hydrogen.

**16.** The use of the molar share of deuterium in downstream compounds based on hydrogen for tracing the origin, especially the energetic origin, of hydrogen bound in the downstream compounds based on hydrogen, wherein the downstream compounds are compounds generated by hydrogenation of carbonyl compounds.

**17.** A process for tracing the origin, especially the energetic origin, of hydrogen bound in downstream compounds based on hydrogen by determining the molar share of deuterium in said downstream compounds based on hydrogen, wherein the downstream compounds are compounds generated by hydrogenation of carbonyl compounds.

Europäisches Patentamt
European Patent Office
Office européen des brevets

# EUROPEAN SEARCH REPORT

Application Number
EP 24 20 7998

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/136097 A1 (SMAJLOVIC IVAN [RS]) 9 June 2011 (2011-06-09) * the whole document * ----- | 16,17 | INV. C25B1/04 C25B15/08 C01B4/00 C07B59/00 C07C29/141 C07C29/145 C07C29/149 C07C209/50 C07D211/00 |
| A | FELLOWS S K ET AL: "Availability of large quantities of low-deuterium hydrogen, and possible uses", INTERNATIONAL JOURNAL OF HYDROGEN ENERGY, ELSEVIER, AMSTERDAM, NL, vol. 6, no. 1, 1 January 1981 (1981-01-01), pages 67-71, XP025591252, ISSN: 0360-3199, DOI: 10.1016/0360-3199(81)90098-7 [retrieved on 1981-01-01] * the whole document * ----- | 16,17 | |
| X | US 2013/274511 A1 (FOLEY PATRICK [US] ET AL) 17 October 2013 (2013-10-17) * the whole document * ----- | 1,2,5-17 | |
| X | RU 1 704 395 C (NIZHNEKAMSKOE PROIZV OB EDINEN [SU]) 10 May 1995 (1995-05-10) * the whole document * ----- | 1-8,11, 14-17 | TECHNICAL FIELDS SEARCHED (IPC) |
| X | WO 2021/089183 A1 (LINDE GMBH [DE]) 14 May 2021 (2021-05-14) * the whole document * ----- | 1-8, 14-17 | C25B C07C C07B C01C C01B |
| A,P | WO 2023/213713 A1 (BASF SE [DE]) 9 November 2023 (2023-11-09) * claims 13, 14 * ----- | 16,17 | |

The present search report has been drawn up for all claims

1

| Place of search | Date of completion of the search | Examiner |
|---|---|---|
| Munich | 5 March 2025 | Ritter, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 20 7998

This annex lists the patent family members relating to the patent documents cited in the above-mentioned European search report. The members are as contained in the European Patent Office EDP file on
The European Patent Office is in no way liable for these particulars which are merely given for the purpose of information.

05-03-2025

| Patent document cited in search report | Publication date | Patent family member(s) | Publication date |
|---|---|---|---|
| US 2011136097 A1 | 09-06-2011 | NONE | |
| US 2013274511 A1 | 17-10-2013 | CA 2867698 A1 | 26-09-2013 |
| | | EP 2828231 A1 | 28-01-2015 |
| | | JP 6510400 B2 | 08-05-2019 |
| | | JP 6591518 B2 | 16-10-2019 |
| | | JP 2015510932 A | 13-04-2015 |
| | | JP 2018052975 A | 05-04-2018 |
| | | US 2013274511 A1 | 17-10-2013 |
| | | WO 2013142206 A1 | 26-09-2013 |
| RU 1704395 C | 10-05-1995 | NONE | |
| WO 2021089183 A1 | 14-05-2021 | DE 102019007672 A1 | 06-05-2021 |
| | | WO 2021089183 A1 | 14-05-2021 |
| WO 2023213713 A1 | 09-11-2023 | AU 2023266064 A1 | 14-11-2024 |
| | | CN 119156370 A | 17-12-2024 |
| | | EP 4519241 A1 | 12-03-2025 |
| | | KR 20250006960 A | 13-01-2025 |
| | | WO 2023213713 A1 | 09-11-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017211784 A
- EP 1317959 A
- DE 10231942
- EP 71787 A
- WO 201760243 A
- US 20110282106 A
- EP 759896 A
- WO 2010115759 A
- DE 10253751
- DE 19750532
- WO 2011061185 A
- EP 3878831 A
- DE 10218848
- WO 2013131936 A
- EP 0759896 A
- WO 2022250790 A
- WO 2020190550 A
- WO 2019158456 A
- DE 10062448
- EP 0987240 A
- EP 358420 A
- US 52319129 B
- US 5399742 A
- US 5387752 A
- EP 696575 A
- WO 9938613 A
- EP 1051388 A
- WO 9938838 A
- US 20090093656 A
- EP 1070698 A
- US 6939995 B
- DE 1228603
- EP 4234665 A1
- DE 19912684 C
- US 20080132738 A
- US 9238602 B
- WO 2011056595 A
- US 20110282109 A
- WO 2010133619 A
- WO 2011133037 A
- WO 2016080552 A
- DE 2513377
- EP 2432756 A
- WO 9305003 A
- US 3729520 A
- EP 24164893
- WO 2007068371 A


### Non-patent literature cited in the description


- **S. KUMAR** ; **V. HIMABINDU**. *Material Science for Energy Technologies*, 2019, vol. 2, 4442-4454
- **H. A. MILLER et al.** *Sustainable Energy Fuels*, 2020, vol. 4, 2114-2133
- **K. HARADA et al.** *International Journal of Hydrogen Energy*, 2020, vol. 45, 31389-31 395
- **H. SATO et al.** *International Journal of Hydrogen Energy*, 2021, vol. 46, 33 689-33 695
- The Carbonyl Group. PATAI'S Chemistry of Functional Groups. John Wiley & Sons, 1966, vol. 1
- The Carbonyl Group. PATAI'S Chemistry of Functional Groups. John Wiley & Sons, 1970, vol. 2
- Hydrogenation of Acids, Esters, Lactones and Anhydrides; Hydrogenation of Aldehydes; Hydrogenation of Ketones. **RYLANDER, P.** Catalytic Hydrogenation in Organic Synthesis. Elsevier, 1979
- **C.STOCK**. Hydrogenation and Dehydrogenation. *Ullmann's Encyclopedia of Industrial Chemistry*, 2023, https://doi.org/10.1002/14356007.a13_487.pub3
- **C. F. H. ALLEN** ; **JAMES VANALLAN**. m-Toylylbenzylamine. *Organic Syntheses.*, 1955, vol. 3, 827
- **B. MEKLER** ; **S. RAMACHANDRAN** ; **S. SWAMINATHAN** ; **MELVIN S. NEWMAN**. 2-Methyl-1,3-Cyclohexanedione. *Organic Syntheses*, 1973, vol. 5, 743
- **C. STOCK**. *Hydrogenation and Dehydrogenation*, 2023
- Catalysts. Ullmann's Encyclopedia of Industrial Chemistry
- **GALLEI, E.F.** ; **NEUMANN, H.-P.** *Chemie Ingenieur Technik*, 1994, vol. 7, 924-928
- **ARMBRÜSTER, M.** *Science and Technology of advanced Materials*, 2020, vol. 21, 303-322
- **ALFA AESAR**. *Platinum Heterogeneous Catalysts*, 18 January 2018, www.alfa.com
- **JIMINEZ-GOMEZ, C.P.** ; **CECILIA, J.A.** ; **DURAN-MARTIN, D. et al.** *J. Catal.*, 2016, vol. 336, 107-115

- **D. STOVER et al.** *Journal of Power Sources*, 15 November 2012, vol. 218, 157-162
- **C.C. KLEPPER** ; **T.M. BIEWER** ; **U. KRUEZI** ; **S. VARTANIAN** ; **D. DOUAI** ; **D.L. HILLIS** ; **C. MARCUS**. Extending helium partial pressure measurement technology to JET DTE2 and ITER. *Rev. Sci. Instrum.*, 2016, vol. 87 (11)
- **S. DAVIES** ; **J.A. REES** ; **D.L. SEYMOUR**. Threshold ionization mass spectrometry (TIMS); A complementary quantitative technique to conventional mass resolved mass spectrometry. *Vacuum*, 2014, vol. 101, 416-422
- **W.A. BRANDT**. *RAPID COMMUNICATIONS IN MASS SPECTROMETRY Rapid Commun. Mass Spectrom.*, 1999, vol. 13, 1226-1230
- SNIF-NMR Part 1: Principles. **GÉRARD J. MARTIN** ; **SERGE AKOKA** ; **MARYVONNE L. MARTIN**. Modern Magnetic Resonance. Springer, 2008, 1651-1658
- SNIF-NMR Part 2: Isotope Ratios as Tracers of Chemical and Biochemical Mechanistic Pathways. **MARYVONNE MARTIN** ; **BENLI ZHANG** ; **GÉRARD J. MARTIN**. Modern Magnetic Resonance. Springer, 2008, 1659-1667
- SNIF-NMR Part 3: From Mechanistic Affiliation to Origin Inference. **GÉRARD J. MARTIN** ; **MARYVONNE L. MARTIN** ; **GÉRALD REMAUD**. Modern Magnetic Resonance. Springer, 2008